# EUROPEAN PATENT APPLICATION

(11) **EP 4 223 171 A1**
(43) Date of publication of application: **09.08.2023**
(21) Application number: 23154643.3
(22) Date of filing: 02.02.2023
(51) Int. Cl.: A41D 13/12, A42B 3/04, A61B 34/00, G02B 7/00

(54) **MIXED REALITY SURGICAL HELMET**

(30) Priority: 02.02.2022 US 202263305750 P; 25.08.2022 US 202263400890 P
(71) Applicant: Zimmer, Inc., Warsaw, IN 46580 (US); Jabil, Inc., St. Petersburg, FL 33716 (US)
(72) Inventor: SPOONER, Ted, Grand Rapids, 49506 (US); COPE, Sean, Warsaw, 46580 (US); MCINTOSH, Kyle, Warsaw, 46580 (US); BREEN, Chris, Clinton, 46580 (US); DUPONT, Alex, Warsaw, 46580 (US); HALL, Ryan, Warsaw, 46580 (US)
(74) Representative: Mays, Julie

(57) **Abstract**

A surgical helmet assembly (200) securable to a mixed reality device (104) and includes an inner frame (204), an outer frame (206), and a surgical hood (210). The inner frame is securable to the mixed reality device. The outer frame is releasably securable to the inner frame, where at least a portion of a lens of the mixed reality device is exposed through the inner frame and the outer frame. The surgical hood is connected to the outer frame and is configured to cover at least a portion of a head of a user.

## Description

### CLAIM OF PRIORITY

This application claims the benefit of U.S. Provisional Patent Application Serial No. 63/305,750, filed on February 2, 2022, and claims the benefit of U.S. Provisional Patent Application Serial No. 63/400,890, filed on August 25, 2022, the benefit of priority of each of which is claimed hereby, and each of which is incorporated by reference herein in its entirety.

### BACKGROUND

Surgical advancements have allowed surgeons to use preoperative planning, display devices within a surgical field, optical imaging, and guides to improve surgical outcomes and customize surgery for a patient. While these advances have allowed for quicker and more successful surgeries, they rely on physical objects, which have costs and time requirements for manufacturing and configuration. Physical objects and devices can also obstruct portions of a surgical field.

Computer-assisted surgery is a growing field that encompasses a wide range of devices, uses, procedures, and computing techniques, such as surgical navigation, pre-operative planning, and various robotic techniques. In computer-assisted surgery procedures, a robotic system can be used in some surgical procedures, such as orthopedic procedures, to aid a surgeon in completing the procedures more accurately, more quickly, or with less fatigue.

### BRIEF DESCRIPTION OF THE DRAWINGS

In the drawings, which are not necessarily drawn to scale, like numerals can describe similar components in different views. Like numerals having different letter suffixes can represent different instances of similar components. The drawings illustrate generally, by way of example, but not by way of limitation, various embodiments discussed in the present document.
FIG. 1 illustrates surgical field in accordance with some embodiments.
FIG. 2 illustrates a perspective view of a surgical helmet assembly.
FIG. 3 illustrates a perspective view of a portion of a surgical helmet assembly.
FIG. 4A illustrates a perspective view of a portion of a surgical helmet assembly.
FIG. 4B illustrates a top perspective view of a portion of a surgical helmet assembly.
FIG. 5A illustrates an isometric view of a surgical helmet assembly.
FIG. 5B illustrates an isometric view of a portion of a surgical helmet assembly.
FIG. 5C illustrates an isometric view of a portion of a surgical helmet assembly.
FIG. 6 illustrates a cross-sectional view of a portion of a surgical helmet assembly.
FIG. 7 illustrates a top perspective view of a portion of a surgical helmet assembly.
FIG. 8 illustrates an isometric view of a portion of a surgical helmet assembly.
FIG. 9 illustrates an isometric view of a portion of a surgical helmet assembly.
FIG. 10 illustrates an isometric view of a portion of a surgical helmet assembly.
FIG. 11 illustrates an isometric view of a portion of a surgical helmet assembly.
FIG. 12 illustrates an isometric view of a portion of a surgical helmet assembly.
FIG. 13 illustrates an isometric view of a portion of a surgical helmet assembly.
FIG. 14A illustrates an isometric view of a portion of a surgical helmet assembly.
FIG. 14B illustrates an isometric view of a portion of a surgical helmet assembly.
FIG. 15A illustrates a perspective view of a portion of a surgical helmet assembly.
FIG. 15B illustrates a perspective view of a portion of a surgical helmet assembly.
FIG. 15C illustrates a perspective view of a portion of a surgical helmet assembly.
FIG. 16 illustrates an isometric view of a portion of a surgical helmet assembly.
FIG. 17A illustrates an isometric view of a portion of a surgical helmet assembly.
FIG. 17B illustrates a cross-sectional side view of a portion of a surgical helmet assembly.
FIG. 18 illustrates a perspective view of a surgical helmet assembly.
FIG. 19 illustrates an enlarged perspective view of a portion of a surgical helmet assembly.
FIG. 20 illustrates an isometric view of a portion of a surgical helmet assembly.
FIG. 21 illustrates an isometric view of a portion of a surgical helmet assembly.
FIG. 22 illustrates an isometric view of a portion of a surgical helmet assembly.

### DETAILED DESCRIPTION

Mixed reality devices can be used during surgeries to help improve surgical performance or to help surgeons or physicians interact with surgical robots (or other devices) during the surgery. These devices can be worn by physicians and at least the lenses of the devices must be exposed to the environment to operate properly. However, mixed reality devices are not easily sterilized.

This disclosure helps to address these issues by providing a surgical helmet assembly to provide protection or covering over the mixed reality device to help minimize exposure of the mixed reality device to the surgical environment. The assembly can interface with a mixed reality device where the assembly can be releasably securable to disposable or cleanable components (such as a visor and a drape) while exposing outer portions of the lenses to the environment to allow the mixed reality device to operate properly. The assembly can include an inner frame securable to the mixed reality device and an outer frame secured to the inner frame where the outer frame can include (or be connected to) a visor and hood. The outer frame can be released from the mixed reality device and inner frame for cleaning or disposal of the outer frame while the inner frame can be hand-sterilized with the lenses of the mixed reality device.

The above discussion is intended to provide an overview of subject matter of the present patent application. It is not intended to provide an exclusive or exhaustive explanation of the invention. The description below is included to provide further information about the present patent application.

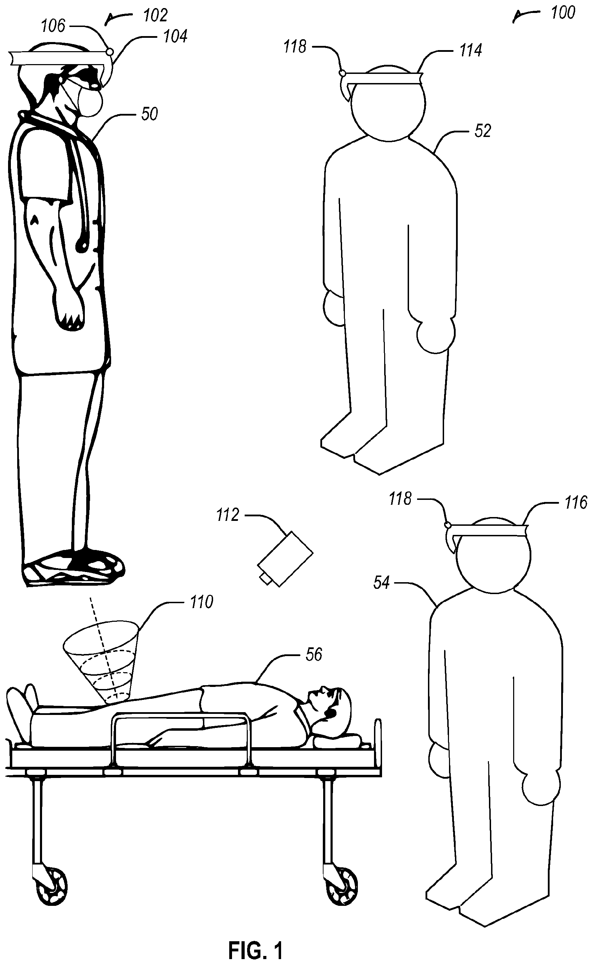

FIG. 1 illustrates a surgical field 100 in accordance with some embodiments. The surgical field 100 illustrated in FIG. 1 can include a surgeon 50, a patient 56, and can include a camera 112. The surgeon 50 can wear a surgical helmet assembly 102 (examples of which are discussed below in further detail) that can include augmented reality (AR) or mixed reality (MR) device 104 which can be used to display a virtual object 110 to the surgeon 50. The virtual object 110 may not be visible to others within the surgical field 100 (e.g., surgical assistant 52 or nurse 54), though they may wear AR or MR devices 114 and 116 respectively (which can include cameras 118 and 120, respectively). Even if another person is viewing the surgical field 100 with an AR or MR device, the person may not be able to see the virtual object 110 or may be able to see the virtual object 110 in a shared augmented reality with the surgeon 50, or may be able to see a modified version of the virtual object 110 (e.g., according to customizations unique to the surgeon 50 or the person) or may see different virtual objects entirely. Augmented reality is explained in more detail below.

Augmented reality is a technology for displaying virtual or "augmented" objects or visual effects overlaid on a real environment. Mixed reality is a similar technology where visual effects are anchored or located relative to real objects in an environment. The disclosure herein can apply to either technology.

The real environment can include a room or specific area (e.g., the surgical field 100), or can be more general to include the world at large. The virtual aspects overlaid on the real environment can be represented as anchored or in a set position relative to one or more aspects of the real environment. For example, the virtual object 110 can be configured to appear to be resting on a table. An AR or MR system can present virtual aspects that are fixed to a real object without regard to a perspective of a viewer or viewers of the system (e.g., the surgeon 50). For example, the virtual object 110 can exist in a room, visible to a viewer of the system within the room and not visible to a viewer of the system outside the room. The virtual object 110 in the room can be displayed to the viewer outside the room when the viewer enters the room. In this example, the room can act as a real object that the virtual object 110 can be fixed to in the system.

The MR device 104 can include one or more screens, such as a single screen or two screens (e.g., one per eye of a user). The screens can allow light to pass through the screens such that aspects of the real environment are visible to the surgeon 50 while displaying the virtual object 110. The virtual object 110 can be made visible to the surgeon 50 by projecting light. The virtual object 110 can appear to have a degree of transparency or can be opaque (i.e., blocking aspects of the real environment).

An MR system can be viewable to one or more viewers, and can include differences among views available for the one or more viewers while retaining some aspects as universal among the views. For example, a heads-up display can change between two views while virtual objects can be fixed to a real object or area in both views. Aspects such as a color of an object, lighting, or other changes can be made among the views without changing a fixed position of at least one virtual object.

A user can see the virtual object 110 presented in an MR system as opaque or as including some level of transparency. In an example, the user can interact with the virtual object 110, such as by moving the virtual object 110 from a first position to a second position. For example, the user can move an object with his or her hand. This can be done in the MR system virtually by determining that the hand has moved into a position coincident or adjacent to the object (e.g., using one or more cameras, which can be mounted on an MR device, such as MR device camera 106 or separate, and which can be static or can be controlled to move), and causing the object to move in response. Virtual aspects can include virtual representations of real world objects or can include visual effects, such as lighting effects, etc. The MR system can include rules to govern the behavior of virtual objects, such as subjecting a virtual object to gravity or friction, or can include other predefined rules that defy real world physical constraints (e.g., floating objects, perpetual motion, etc.). An MR device 104 can include a camera 106 (not to be confused with the camera 112, separate from the MR device 104). The MR device camera 106 or the camera 112 can include an infrared camera, an infrared filter, a visible light filter, a plurality of cameras, a depth camera, etc. The MR device 104 can project virtual items over a representation of a real environment, which can be viewed by a user.

Eye tracking can be used with a MR system to determine which instrument a surgeon wants next by tracking the surgeon's eye to the instrument. In an example, a nurse or surgical assistant can then retrieve the determined instrument. The determined instrument can be presented in MR to the nurse or surgical assistant. In another example, the surgeon can speak the instrument (e.g., using a pre-selected code word, using speech processing and word recognition, via saying a number, or the like). The voice command can be combined with eye tracking, in still another example, to find an instrument.

The MR device 104 can be used in the surgical field 100 during a surgical procedure, for example performed by the surgeon 50 on the patient 56. The MR device 104 can project or display virtual objects, such as the virtual object 110 during the surgical procedure to augment the surgeon's vision. The surgeon 50 can control the virtual object 110 using the MR device 104, a remote controller for the MR device 104, or by interacting with the virtual object 110 (e.g., using a hand to "interact" with the virtual object 110 or a gesture recognized by the camera 106 of the MR device 104). The virtual object 108 can augment a surgical tool. For example, the virtual object 110 can appear (to the surgeon 50 viewing the virtual object 110 through the MR device 104) as a representation of a landmark previously placed on a patient bone. In another example, the virtual object 110 can be used to represent a planned location of a landmark (e.g., using a pre-operative image and a captured image of the bone in the real space). In certain examples, the virtual object 110 can react to movements of other virtual or real-world objects in the surgical field. For example, the virtual object 110 can be altered by a to move a landmark (e.g., a placed landmark). In other examples, the virtual object 110 can be a virtual representation of a remote surgical field (e.g., an entire OR, a camera field of view of a room, a close-up view of a surgical theater, etc.). In this example, the virtual object 110 can include a plurality of virtual objects.

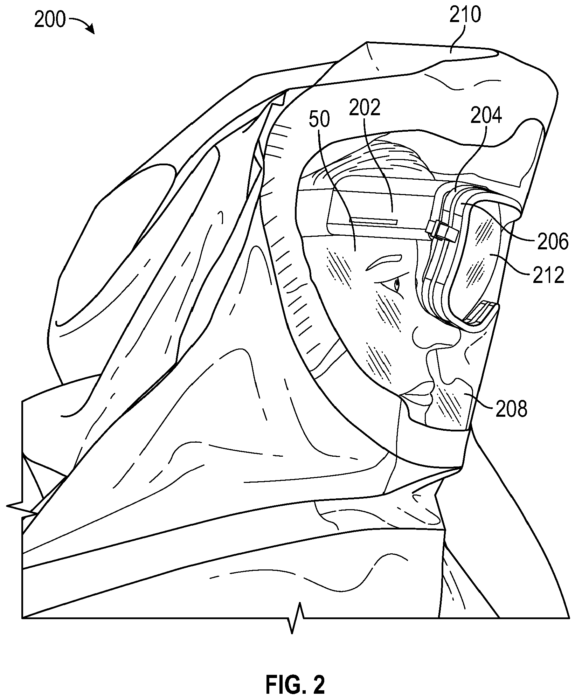

FIG. 2 illustrates a perspective view of a surgical helmet assembly 200, which can be similar to the surgical helmet 102 discussed above. The surgical helmet assembly 200 is discussed in greater detail below. FIG. 2 also shows a user 50 or surgeon.

The surgical helmet assembly 200 can include an MR device 202, which can be similar to any of the AR or MR devices discussed above. The MR device 202 can be securable to a head of the surgeon 50. The surgical helmet assembly 200 can also include an inner frame 204 securable to the MR device 202 and the surgical helmet assembly 200 can include an outer frame 206. The surgical helmet assembly 200 can also include a visor 208 and hood 210.

The visor 208 can be a transparent or translucent shield configured to provide visibility for the surgeon 50 while limiting transfer of matter between the surgeon 50 and the surgical environment. The hood 210 can be a drape or covering connected to the visor 208. The hood 210 can be secured to a perimeter of the visor 208 or the outer frame 206 and can be configured to cover at least a portion of a head of the surgeon 50. The outer frame 206 can be secured to the visor 208 or the hood 210. The outer frame 206 can be releasably securable to the inner frame 204 to enclose at least a portion of the head and neck of the surgeon 50 in the surgical helmet assembly 200 and so that at least a portion of a lens 212 of the MR device 202 is exposed to the surgical environment through the inner frame 204 and the outer frame 26.

In operation, the surgeon 50 can wear the MR device 202 with the inner frame 204 attached thereto. Then, the outer frame 206 can be secured to the inner frame 204 to secure the visor 208 and the hood 210 to the MR device 202 and to enclose the head of the surgeon 50, but exposing the lens 212 to the environment to allow the lens 212 to operate normally. Following a procedure or other use of the MR device 202, the outer frame 206, the visor 208, and the hood 210 can be removed from the inner frame 204, such as by disengaging the inner frame 204 from the outer frame 206. In some examples, the surgical helmet assembly 200 can be configured such that the lens 212 is the only portion of the MR device 202 exposed to the environment, which can allow the MR device 202 to be sterilized and reused while a remainder of the surgical helmet assembly 200 (such as the outer frame 206, the visor 208, and the hood 210) can be disposed or otherwise sterilized. Further details of the surgical helmet assembly 200 are discussed below.

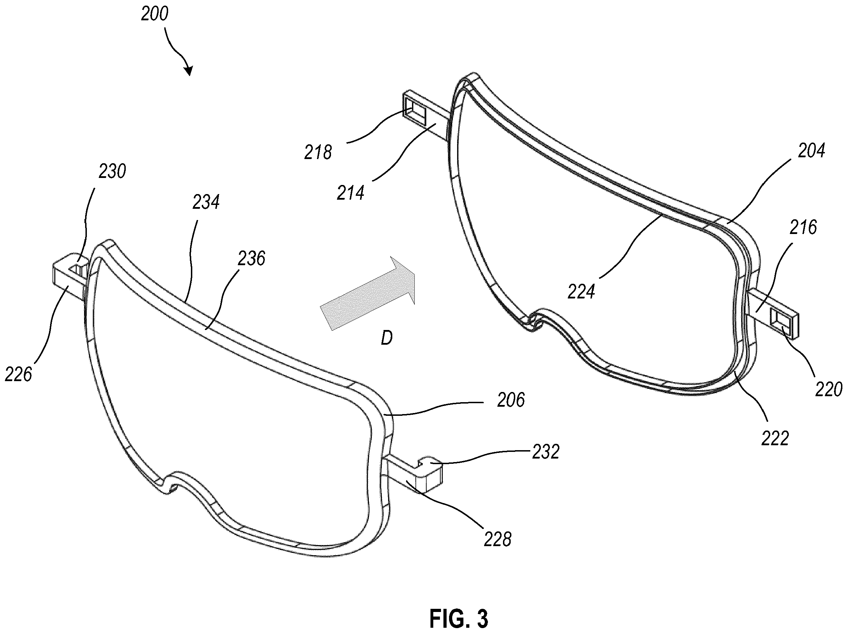

FIG. 3 illustrates a perspective view of the inner frame 204 and the outer frame 206 of the surgical helmet assembly 200. The surgical helmet assembly 200 can be consistent with the surgical helmet assembly 200 of FIG. 2. FIG. 3 shows additional details of the frames.

For example, FIG. 3 shows that the surgical helmet assembly 200 can include a first arm 214 and a second arm 216 where the first arm 214 can extend laterally from the inner frame 204 and the second arm 216 can extend laterally from the inner frame 204 opposite the first arm 214. The first arm 214 can include a first bore or slot 218 extending therethrough and the second arm 216 can include a second bore or slot 220 extending therethrough. The inner frame 204 can also define or include a channel 222 extending around a periphery of an outer surface 224 of the inner frame 204. The channel 222 can be configured to support a gasket or seal therein, as discussed in further detail below.

The surgical helmet assembly 200 can include a third arm 226 extending from the outer frame 206 and can include a fourth arm 228 extending from the outer frame 206 opposite the third arm 226. The third arm 226 can include a projection 230 extending perpendicular to the third arm 226 and optionally back towards the outer frame 206, such as to form a J-shape or a U-shape. Similarly, the fourth arm 228 can include a projection 232 extending perpendicular to the fourth arm 228 and optionally back towards the outer frame 206, such as to form a J-shape or a U-shape.

In operation, when the outer frame 206 is moved toward the inner frame 204, such as in the direction D, the first arm 214 can be securable to the third arm 226 by the projection 230 at least partially inserting into the slot 218. Similarly, the second arm 216 can be securable to the fourth arm 228 by the projection 232 at least partially inserting into the slot 220 to secure the inner frame 204 to the outer frame 206. When the outer frame 206 is secured to the inner frame 204 an inner surface 234 of the outer frame 206 can engage the gasket or seal supported by the channel 222 of the outer surface 224 of the inner frame 204 to form a seal between the inner frame 204 and the outer frame 206 such that an exterior surface 236 of the outer frame 206 becomes an outer surface of the frames, as discussed in further detail below. The inner surface 234 can be shaped to be complimentary to the outer surface 224 of the inner frame 204 such that the inner frame 204 mates with the outer frame 206. Though the inner frame 204 and the outer frame 206 are shown as being curved, the inner frame 204 can have a relatively larger thickness such that the outer surface 224 is relatively flat or planar and the inner surface 234 can be shaped complimentary (flat or planar) to help improve sealing between the inner frame 204 and the outer frame 206.

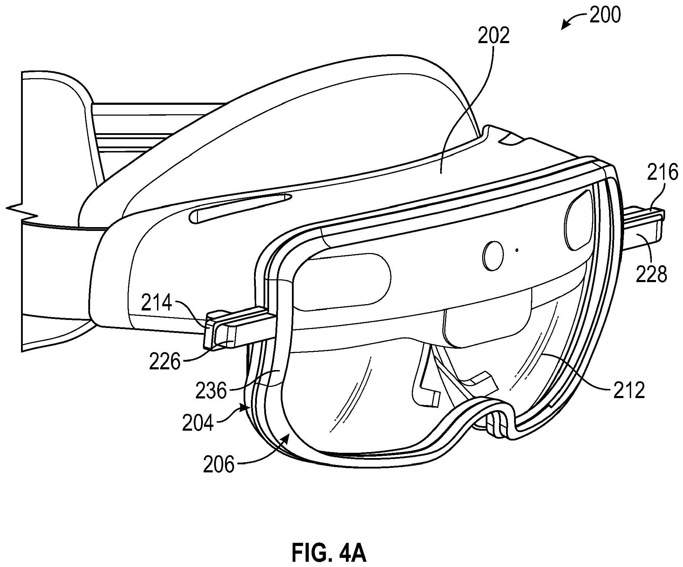

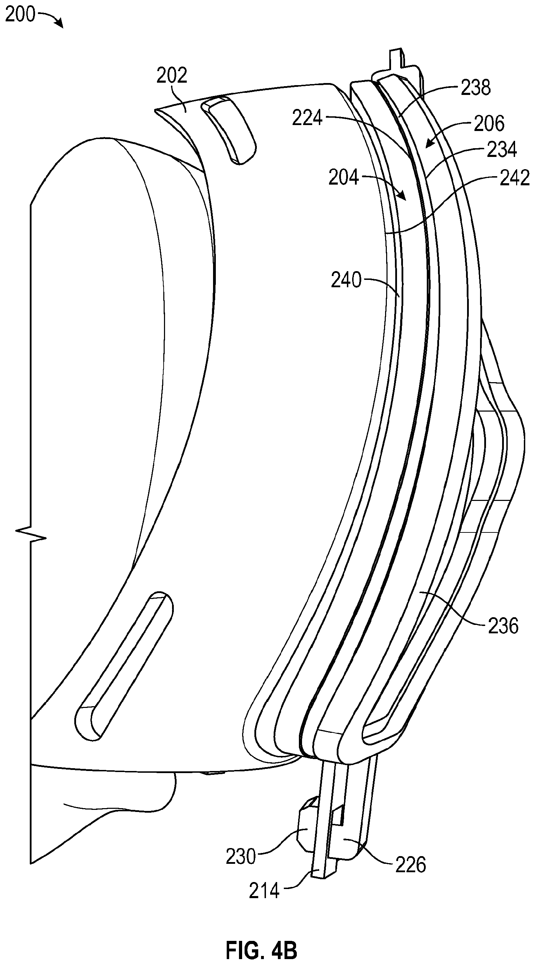

FIG. 4A illustrates a perspective view of a portion of the surgical helmet assembly 200. FIG. 4B illustrates a top perspective view of a portion of the surgical helmet assembly 200. FIGS. 4A and 4B are discussed together below. The surgical helmet assembly 200 can be consistent with FIGS. 2 and 3, FIGS. 4A and 4B show additional details of the surgical helmet assembly 200.

For example, FIG. 4A shows that when the inner frame 204 and the outer frame 206 are secured to each other, the lens 212 of the MR device 202 is exposed through the inner frame 204 and the outer frame 206 to the environment to allow the MR device 202 to operate or function normally without visual interference.

Also, FIG. 4B shows that the surgical helmet assembly 200 can include a gasket 238 engageable with the outer surface 224 of the inner frame 204 and engageable with the inner surface 234 of the outer frame 206 to form a seal between the inner frame 204 and the outer frame 206 when the first and second arms 214 and 216 are secured to the third and fourth arms 226 and 228. The gasket 238 can be compressible between the inner frame 204 and the outer frame 206 when the arms are connected to help ensure a seal is formed between the inner frame 204 and the outer frame 206. Optionally, the gasket 238 can be secured to the outer frame 206 and engageable with the inner frame 204 such that the gasket 238 is disposed of between uses whereas the inner frame 204 can be reused. Optionally, the gasket 238 can be reusable.

FIG. 4B also shows that an inner surface 240 of the inner frame 204 can be engageable with and securable to an outer surface 242 of the MR device 202 such as to form a seal between the inner frame 204 and the MR device 202. The inner surface 240 can be secured to the outer surface 242 using one or more of an adhesive, screws, rivets, hook and loop fasteners, or the like. FIG. 4B also shows that the exterior surface 236 of the outer frame 206 can be the outer surface of the frames when the inner frame 204 is connected to the outer frame 206. The exterior surface 236 can receive an adhesive or other fastener to secure the exterior surface 236 to the visor 208 or the hood 210.

FIG. 4B also shows how the inner frame 204 can be secured to the outer frame 206. When the projection 230 is inserted through the slot 218 of the first arm 214, the projection 230 can extend laterally inward (toward the second arm 216) to engage a rear portion of the first arm 214 such as to help limit separation between the inner frame 204 and the outer frame 206 and to help keep the gasket 238 compressed between the inner frame 204 and the outer frame 206.

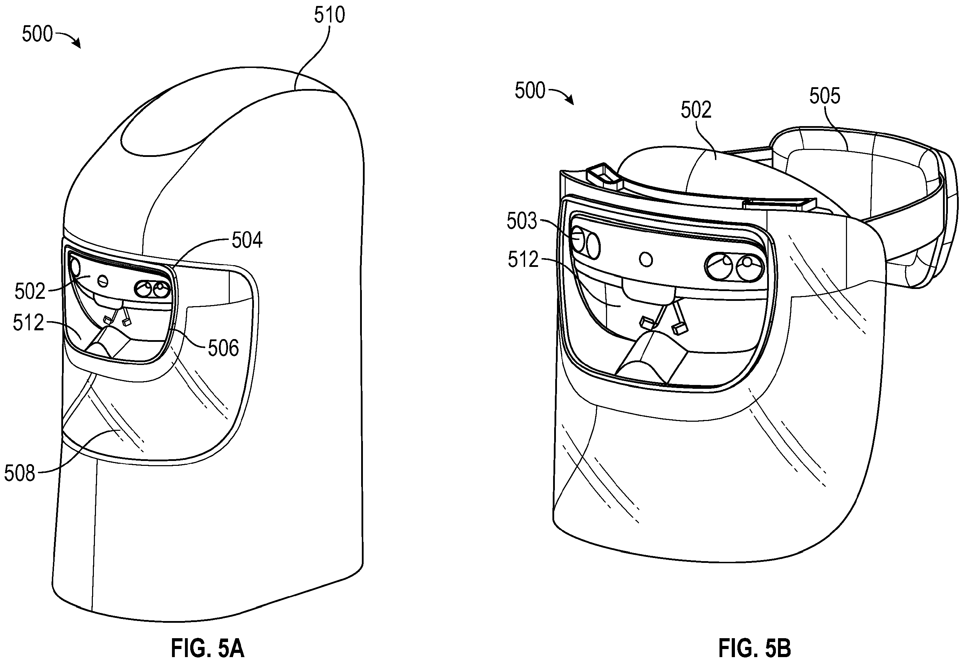

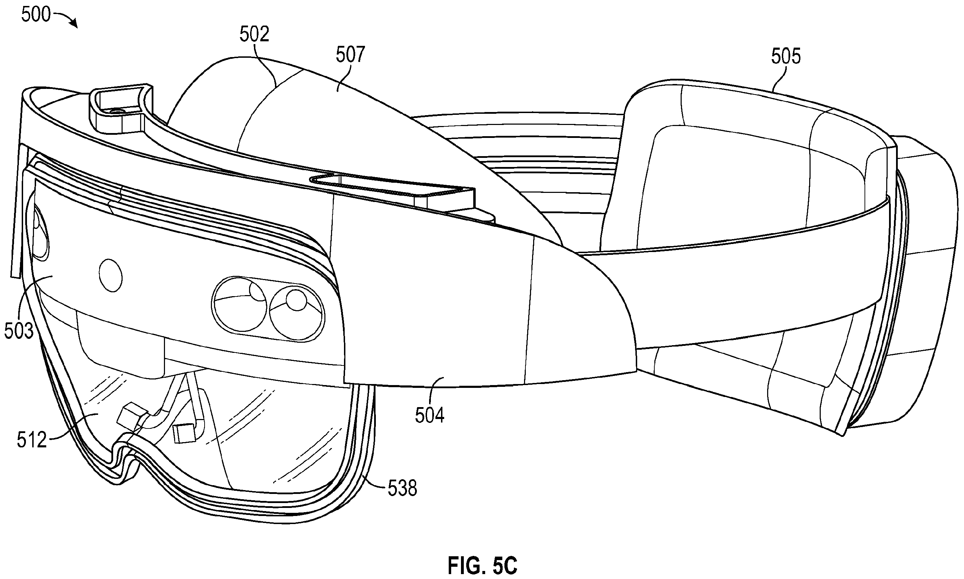

FIG. 5A illustrates an isometric view of a surgical helmet assembly 500. FIG. 5B illustrates an isometric view of a portion of the surgical helmet assembly 500. FIG. 5C illustrates an isometric view of a portion of the surgical helmet assembly 500. FIGS. 5A-5C are discussed together below. The surgical helmet assembly 500 can be similar to the surgical helmet assembly 200 discussed above; the surgical helmet assembly 500 can differ in that the outer frame can include the visor and the inner frame can be secured to the AR or MR device. Any of the helmet assemblies discussed above or below can be modified to include the components of the surgical helmet assembly 500. Further details are discussed below.

The surgical helmet assembly 500 can include an MR device 502, which can be similar to those discussed above and can include a sensor array 503, a battery pack 505, and a body 507. The body 507 can support the lenses 512 and other components of the MR device 502. An inner frame 504 can be a polymer member releasably securable to one or more components of the MR device 502, such as the body 507. An outer frame 506 can include a visor 508 and a hood 510 and the outer frame 506 can be releasably securable to the inner frame 504, such as through one or more of hook and loop fasteners, clamps, clips, temporary adhesive, or the like. The hood 510 can be configured to at least partially surround a head of the surgeon and the visor 508 can be oriented in front of a face of the user to help increase peripheral visibility. The hood 510 can be secured to the visor 508 and the hood 510 and the visor 508 can be disposable or sterilizable following removal from the inner frame 504.

FIG. 5C shows that the MR device 502 can include a seal or gasket 538 surrounding at least a portion of a periphery of the lenses 512. The inner frame 504 can engage the gasket or seal 538 connected to the lenses 512 such as to form a seal between the inner frame 504 and the lenses 512 (and the sensor array 503). The visor 508 can also engage the seal 538 to form a seal between the visor 508 and the lenses 512. The surgical helmet assembly 500 can optionally be modified to include the inner frame 204 of the surgical helmet assembly 200. Further details are discussed with reference to the figures below.

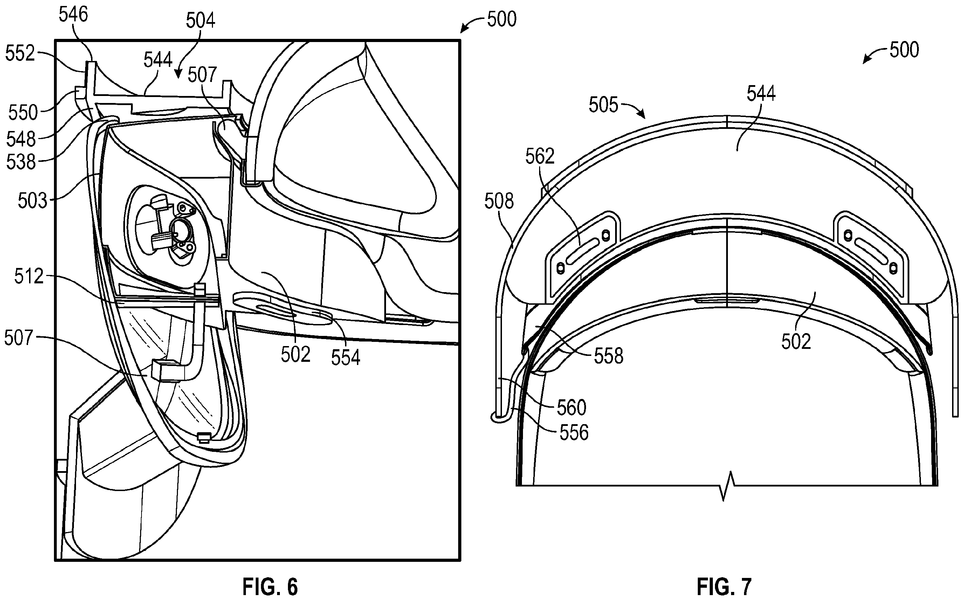

FIG. 6 illustrates a cross-sectional view of a portion of the surgical helmet assembly 500. The surgical helmet assembly 500 of FIG. 6 can be consistent with the surgical helmet assembly 500 discussed above with respect to FIGS. 5A-5C. FIG. 6 shows additional details of the surgical helmet assembly 500.

For example, FIG. 6 shows that the inner frame 504 can include a top cover 544, a top surface 546, a sealing portion 548, and a lip 550. The top cover 544 can be securable to the body 507 of the MR device 502 as discussed below in further detail. The top surface or portion 546 can be connected to the top cover 544 and can be configured to receive and be secured to a top portion 552 of the visor 508 such as to form a seal between the inner frame 504 and the visor 508 above the lenses 512 and the sensor array 503.

The lip 550 can be connected to the top surface 546 and can be located below the top surface 546 and the top portion 552. The lip 550 can extend laterally beyond the top portion 552 such as to catch fluids or debris from the environment and guide them away from the seal formed between the top portion 552 and the top surface 546.

The sealing portion 548 can be a portion of the inner frame 504 extending downward from the top cover 544 and optionally toward the sensor array 503. The sealing portion 548 can be engageable with the seal 538 such as to compress the seal 538 between the sealing portion 548 and the sensor array 503 or the lenses 512. FIG. 6 also shows a pillar 554 that can be used to connect the inner frame 504 to the MR device 502, as discussed in further detail below.

FIG. 7 illustrates a top perspective view of a portion of the surgical helmet assembly 500. The surgical helmet assembly 500 of FIG. 7 can be consistent with the surgical helmet assembly 500 discussed above with respect to FIGS. 5A-6. FIG. 7 shows additional details of the surgical helmet assembly 500. For example, FIG. 7 shows that the surgical helmet assembly 500 can include a clip 556 securable to a flange 558 of the MR device 502 and to a rear portion 560 of the visor. Such a clip can be a clamp, fastener, or the like configured to further secure the visor 508 and the hood 510 to the inner frame 504 and therefore to the MR device 502. FIG. 7 also shows mounting provisions 562 in the top cover 544 for the pillar 554, as discussed in further detail below.

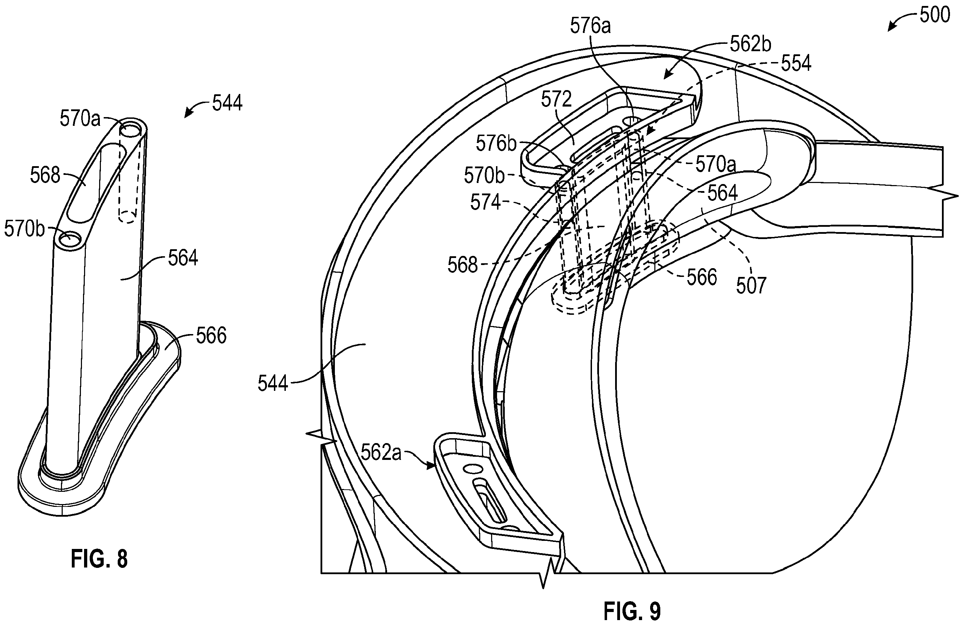

FIG. 8 illustrates an isometric view of the pillar 554 of the surgical helmet assembly 500. The pillar 554 can include a body 564 that can be curved to match a bore or slot of the body 507 of the MR device 502. The pillar 554 can also include a flange 566 connected to a bottom portion of the body 564. The body 564 can define a cooling channel 568, which can be a bore, slot, or channel extending through the body 564 and the flange 566. The body 564 can also include a bore 570a and a bore 570b, which can be bores extending at least partially into the body 564 from a top portion of the body 564 on either or opposing sides of the cooling channel 568. The bore 570a and the bore 570b can each be threaded such as to receive a fastener (e.g., a bolt or a screw) therein. As discussed in further detail below, the pillar 554 can be at least partially insertable into a portion of the mixed reality device 502 and can be engageable with the inner frame 504 to secure the inner frame 504 to the mixed reality device 502.

FIG. 9 illustrates an isometric view of a portion of the surgical helmet assembly 500, which can be consistent with the surgical helmet assembly 500 discussed in FIGS. 5A-8 above. FIG. 9 shows additional details of how the MR device 502 can be secured to the inner frame 504. For example, FIG. 9 shows that the top cover 544 can include a mount 562a and a mount 562b, which can each be configured to be secured to a pillar (e.g., the pillar 554) to secure the inner frame 504 to the MR device 502.

The mounts 562a and 562b can each include a cooling port 572 alignable with the cooling channel 568 when the pillar 554 is inserted into a cooling channel 574 of the body 507 of the MR device 502. The mount 562a and the mount 562b can also each include a bore 576a and a bore 576b, where the bore 576a can be alignable with the bore 570a and the bore 576b can be alignable with the bore 570b when the pillar 554 is inserted into the cooling channel 574 of the body 507 of the MR device 502.

When the pillar 554 is inserted into the cooling channel 574, the body 564 of the pillar 554 can extend substantially through the cooling channel 574 and the flange 566 can engage the body 507 of the MR device 502 to limit upward movement of the pillar 554 with respect to the body 507 and the cooling channel 574. Also, when the pillar 554 is fully inserted into the cooling channel 574, the bore 576a and the bore 576b can be aligned with the bore 570a and the bore 570b, respectively, such that the bore 576a and the bore 576b can each receive fasteners therein to extend into the bore 570a and the bore 570b. Securing or tightening of the fasteners into the bores can cause the flange 566 to, together with the fasteners, clamp the pillar 554 to the top cover 544 and the body 507 of the MR device 502 helping to secure the inner frame 504 to the body 507 of the MR device 502.

When the pillar 554 is positioned within the cooling channel 574, the cooling port 572 and the cooling channel 568 can be aligned to transmit air, such as cooling air, from a cooling system to the face of the user or along the visor to help keep the user cool during operation of the MR device 502 and the help limit fogging of the visor 508 and the lenses 512 during use of the MR device 502.

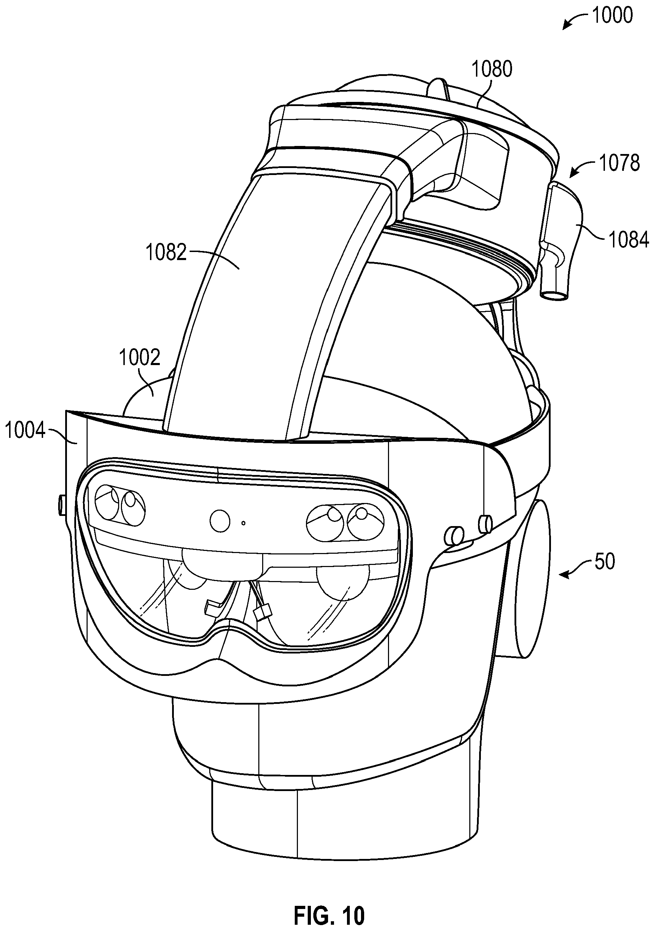

FIG. 10 illustrates an isometric view of a portion of a surgical helmet assembly 1000. The surgical helmet assembly 1000 can be similar to the surgical helmet assemblies discussed above. The surgical helmet assembly 1000 can include a cooling system. Any of the surgical helmet assemblies discussed above or below can include such a system.

The surgical helmet assembly 1000 can include a cooling system 1078 connected to an inner frame 1004 and connectable to the mixed reality device 1002. The 1078 can generally be locatable or located within the surgical hood. Generally, the cooling system 1078 can be operable to deliver conditioned air to the inner frame 1004, the mixed reality device 1002, and the surgical hood.

Optionally, the cooling system 1078 can include a fan or blower 1080 connected to a duct or plenum 1082 (downstream) and air inlets 1084 (upstream). The blower 1080 can be operated to draw air through the air inlets 1084 and the blower 1080 to discharge air through the plenum 1082 and to the inner frame 1004 and the mixed reality device 1002, such as through the pillars, as discussed above, to help cool the user 50 and to help limit fogging of the visor and lenses of the mixed reality device 1002. The cooling system 1078 can optionally include other cooling components, such as an active cooling or heating system (e.g., refrigeration, chilled water, thermoelectric (Peltier) cooler, or the like) that can optionally include one or more heat exchangers.

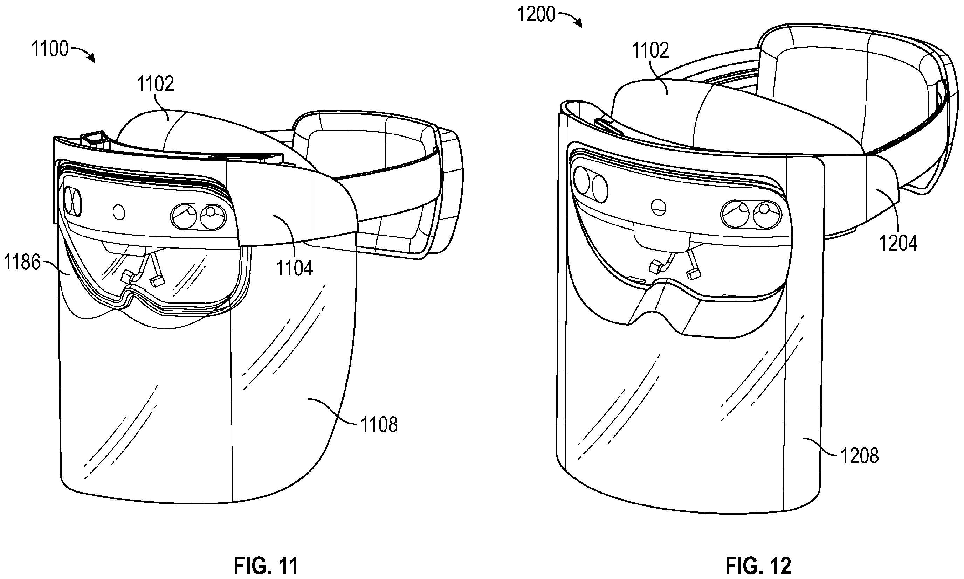

FIG. 11 illustrates an isometric view of a portion of a surgical helmet assembly 1100. The surgical helmet assembly 1100 can be similar to the surgical helmet assemblies discussed above. The surgical helmet assembly 1100 can include a smaller visor. Any of the surgical helmet assemblies discussed above or below can include such a visor.

More specifically, a visor 1108 of the surgical helmet assembly 1100 can be connectable to an inner frame 1104 of the surgical helmet assembly 1100 such that the visor 1108 forms a seal with a lower portion of lenses of an MR device 1102 and the inner frame 1104 forms a seal with an upper portion of the lenses. The visor 1108 can also include a transparent nosepiece that can be integrated to the visor 1108, which can help to improve visibility of a user.

FIG. 12 illustrates an isometric view of a portion of a surgical helmet assembly 1200. The surgical helmet assembly 1200 can be similar to the surgical helmet assemblies discussed above. The surgical helmet assembly 1200 can include a visor 1208 that extends only partially rearward to cover a portion of an inner frame 1204 such that a hood covers the remainder of the inner frame 1204. Any of the surgical helmet assemblies discussed above or below can include such a visor.

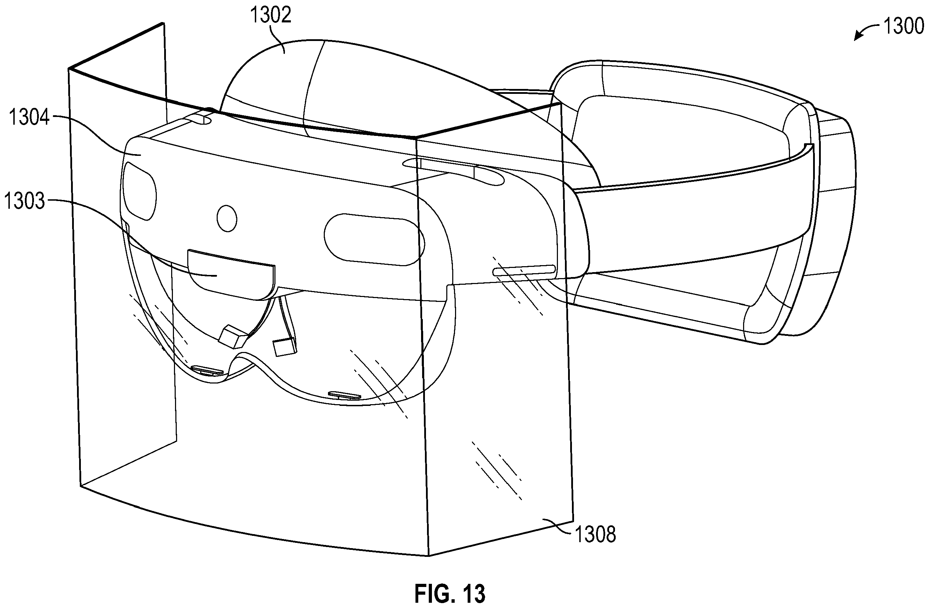

FIG. 13 illustrates an isometric view of a portion of a surgical helmet assembly 1300. The surgical helmet assembly 1300 can be similar to the surgical helmet assemblies discussed above. The surgical helmet assembly 1300 can differ in that the surgical helmet assembly 1300 can include a sensor array that is not exposed through the visor. Any of the surgical helmet assemblies discussed above or below can be modified to include such a visor.

More specifically, the surgical helmet assembly 1300 can include an MR device 1302 (including a sensor array 1303), an inner frame 1304, and a visor 1308. The visor 1308 can be connected to the inner frame 1304 and can partially or entirely cover the sensor array 1303, which can limit exposure of the sensor array 1303 to the surgical field during a procedure. Because of this, the sensor array 1303 does not necessarily require sterilization following a procedure, which can help prolong a life of the device.

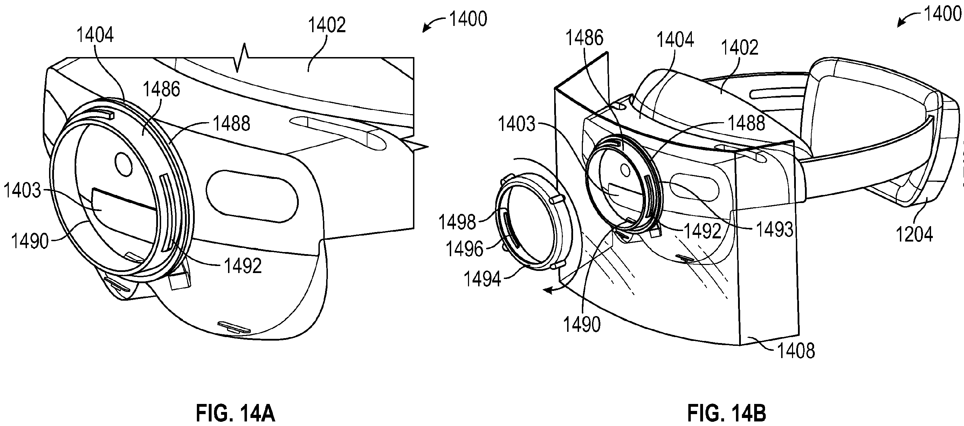

FIG. 14A illustrates an isometric view of a portion of a surgical helmet assembly 1400. FIG. 14B illustrates an isometric view of a portion of the surgical helmet assembly 1400. FIGS. 14A-14B are discussed together below. The surgical helmet assembly 1400 can be similar to the surgical helmet assemblies discussed above. The surgical helmet assembly 1400 can differ in that the surgical helmet assembly 1400 can include a first frame and a second frame configured to expose the sensor array of the surgical helmet assembly 1400 to the surgical field while limiting exposure of other components to the surgical field. Any of the surgical helmet assemblies discussed above or below can be modified to include such a first frame and second frame.

More specifically, the surgical helmet assembly 1400 can include an MR device 1402 (which can include a sensor array 1403), a first frame 1404, and a second frame 1408. The first frame 1404 can be a frame that is connected to the MR device 1402. In some examples, the first frame 1404 can connect to an upper portion of the MR device 1402 and can include a ring 1486. The ring 1486 can be a cylindrical frame member made of one or more of foam, polymer, metal, or the like. The ring 1486 can have other shapes in other examples. In some examples, the first frame 1404 can include only the ring 1486 such that the ring 1486 is secured directly to the MR device 1402. In other examples, the first frame 1404 can include other components secured to the MR device 1402. The ring 1486 can include a gasket 1488 located thereon or therearound.

The ring 1486 can be configured to at least partially surround the sensor array 1403 such that the sensor array 1403 is located within an opening 1490 of the of the ring 1486. The ring 1486 can optionally include threading 1492 on an outer surface of the ring 1486. The second frame 1408 can be or can include a visor and can define a bore 1493 therein or therethrough. The bore 1493 can be positionable around the ring 1486 to allow the second frame 1408 to engage and cover the first frame 1404. In such an orientation the second frame 1408 can cover most of the MR device 1402.

The surgical helmet assembly 1400 can also include a fastener 1494, which can be, for example, a lock ring. The fastener 1494 can include threading 1496 on a radially inner surface thereof and can define an opening 1498 therein or therethrough. The fastener 1494 can be threadably securable to the ring 1486 such as via the threading 1494 and threading 1492. When the fastener 1494 is secured to the ring 1486, the gasket 1488 can be compressed between the ring 1486 and the fastener 1494 such as to form a seal between the ring 1486 and the fastener 1494 and therefore between the first frame 1404 and the second frame 1408. In this way, the second frame 1408 can be releasably securable to the first frame 1404, as discussed in further detail below.

When the fastener 1494 is secured to the ring 1486, the opening 1498 of the fastener 1494 can be aligned with the opening 1490 of the ring 1486 to allow the sensor array 1403 to be exposed to the surgical environment through the first frame 1404 and the second frame 1408, helping to limit interference with the sensor array 1403 caused by the surgical helmet assembly 1400, such as during a procedure using the MR device 1402. The opening 1490 and the opening 1498 can both be circular openings, but can have other shapes in other examples, such as to minimize exposure of the MR device 1402 to the environment while still exposing the sensor array 1403 to the environment.

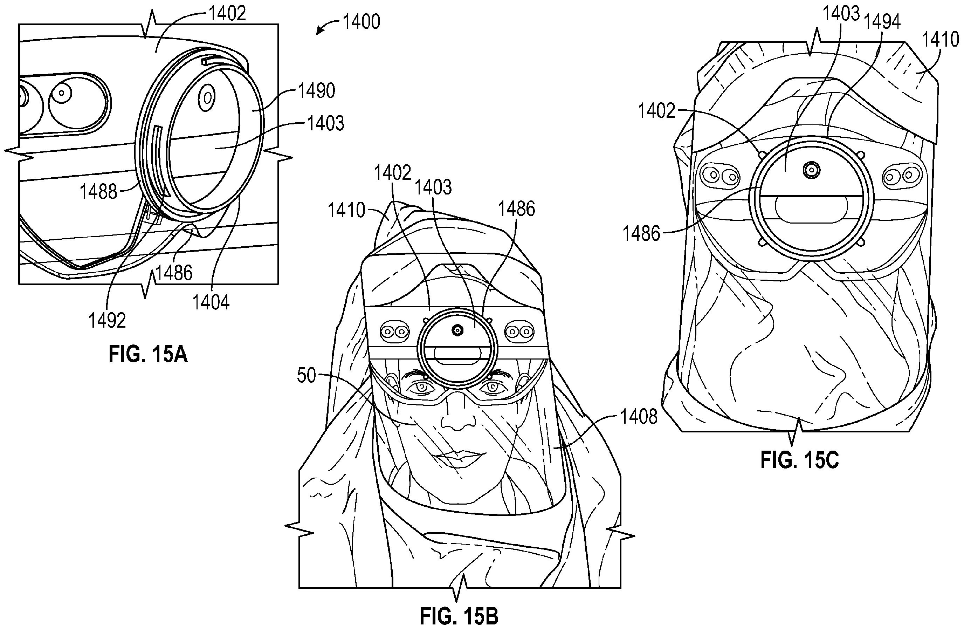

FIG. 15A illustrates a perspective view of a portion of the surgical helmet assembly 1400. FIG. 15B illustrates a perspective view of a portion of the surgical helmet assembly 1400. FIG. 15C illustrates a perspective view of a portion of the surgical helmet assembly 1400. FIGS. 15A-15C are discussed together below. The surgical helmet assembly 1400 can be consistent with the surgical helmet assembly 1400 discussed above with respect to FIGS. 14A-14B; FIGS. 15A-15C show how the surgical helmet assembly 1400 can be used.

For example, FIG. 15A shows that the first frame 1404 can include just the ring 1486 secured to the MR device 1402 with the gasket 1488 installed or secured to the ring 1486. In such a configuration, the opening 1490 of the ring 1486 can expose the sensor array 1403 of the MR device 1402 to the surgical field. Then, as shown in FIG. 15B, the second frame 1408 can be positioned around the ring 1486 to connect the second frame 1408 to the first frame 1404 while still exposing the sensor array 1403 through the second frame 1408. A hood 1410 can be secured to the second frame 1408 such as to enclose the surgeon 50. Then, as shown in FIG. 15C, the fastener 1494 can be secured to the ring 1486 to form a seal between the ring 1486, the fastener 1494, and the second frame 1408, leaving only the sensor array 1403 exposed to the surgical field, which can help to enclose the user 50 and can help to limit exposure of the MR device 1402 to the surgical environment while still exposing the sensor array 1403 to the surgical environment such as to allow the MR device 1402 to operate normally during a surgical procedure.

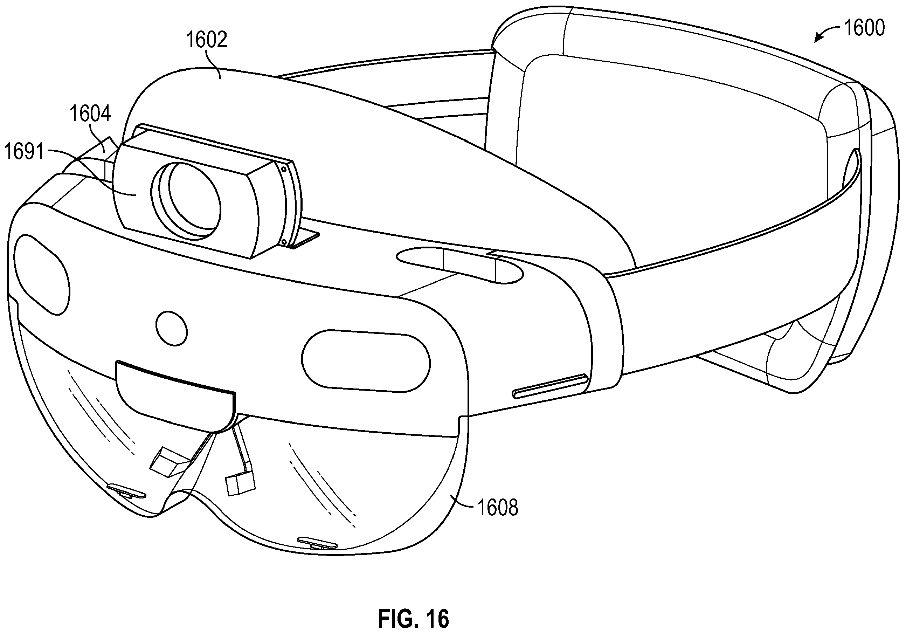

FIG. 16 illustrates an isometric view of a portion of a surgical helmet assembly. The surgical helmet assembly 1600 can be similar to the surgical helmet assemblies discussed above. The surgical helmet assembly 1600 can differ in that the surgical helmet assembly 1600 can include a light 1691 secured to a frame 1604 or an MR device 1602. Any of the surgical helmet assemblies discussed above or below can be modified to include such a light.

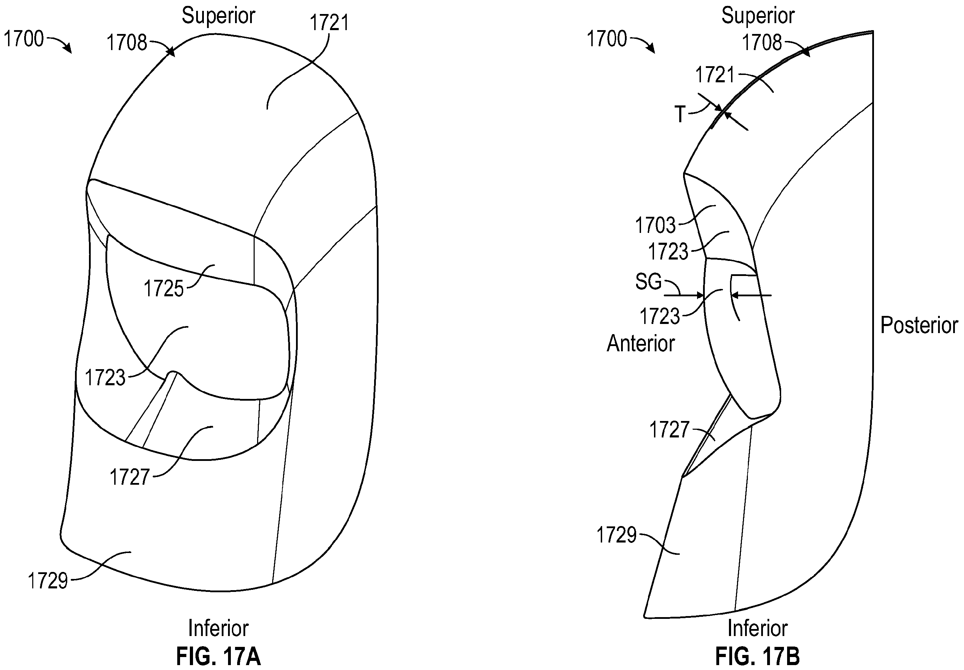

FIG. 17A illustrates an isometric view of a visor 1708 of a surgical helmet assembly 1700. FIG. 17B illustrates a cross-sectional side view of the visor 1708 of a surgical helmet assembly 1700. FIGS. 17A and 17B are discussed together below. The visor 1708 can be similar to those discussed above; the visor 1700 can be formed to support a surgical hood and can be secured to an MR device. Any of the surgical helmet assemblies discussed above or below can be modified to include the features of the surgical helmet assembly 1700.

The visor 1708 can be a rigid or semi-rigid and transparent or translucent shield configured to provide visibility for the surgeon 50 while limiting transfer of matter between the surgeon 50 and the surgical environment. The visor 1708 can be releasably securable to an MR device, where at least a portion of a lens (or sensor array) of the MR device can be spaced away from the visor. Similar to the surgical helmet assemblies discussed above, a surgical hood can be connected to the visor 1708 and can be configured to cover at least a portion of a head of a user and to enclose at least a portion of the MR device. The visor 1708 can optionally be formed as a single piece or as a unitary piece or from a single piece. The visor 1708 can have a thickness T between 0.5 and 1.0 millimeters. Optionally, the thickness T can be about 0.75 millimeters. The thickness T can help to provide structural rigidity of the visor 1708 to provide sufficient rigidity of the visor 1708 while minimizing weight thereof.

The visor 1708 can include an upper portion 1721 that can be shaped, such as curved (or otherwise dimensioned) to form an air gap between a top portion of a user's head and the visor and the surgical hood. The upper portion 1721 can be connected to a sensor portion 1723 (such as via an angled portion 1725) and the upper portion 1721 can extend superiorly and posteriorly therefrom. The upper portion 1721 can be curved from anterior to posterior as the visor extends superiorly from the sensor portion 1723 to form the air gap. The angled portion 1725 can connect the sensor portion 1723 and the upper portion 1721, the angled portion 1725 can be angled with respect to the sensor portion to help transmit light from the inside of the toga, such as the light 1691, to the exterior of the toga to help improve a viewing field of the user and can help direct light away from the sensor portion 1723.

The sensor portion 1723 can also be configured to mate with a lens portion of the MR device and can be configured (e.g., sized and shaped) to allow the user to look through the lenses of the MR device and the sensor portion 1723. The sensor portion 1723 can also be configured to have a shape that is complementary to a shape of the visor or lens of the MR device. In other words, a gap between the sensor portion 1723 and the MR device can be constant.

The visor 1708, e.g., any portion thereof, can be engageable with the MR device to position sensors of the MR device away from the sensor portion 1723 of the visor by a distance of a sensor gap SG, which can help the MR device to operate normally when connected to the surgical helmet assembly 1700. For example, covering or blocking the sensors of the MR device can interfere with the ability of the device to map the environment around the user and perform other functions. Maintaining the proper sensor gap can allow the sensors and camera (of the MR device) to operate as they would without any covering.

Optionally, the sensor gap SG can be between 0.1 millimeters and 5 millimeters. Optionally, the sensor gap SG can be between 0.5 millimeter and 2 millimeters. Optionally, the sensor gap SG can be about 1 millimeter.

The visor 1708 can also include a lower angled portion 1727 that can be connected to the sensor portion 1723. The lower angled portion 1727 can be configured (e.g., sized and shaped) such that the curvature and dimensions create a gap between a face of the user and the visor 1708. The visor 1708 can also include a lower portion 1729 connected to the lower angled portion 1727 and extending inferiorly therefrom. The lower portion 1729 can be sized and shaped to provide mouth visibility for others and can help improve downward visibility around the hood for the user. The lower angled portion 1727 and the lower portion 1729 can also be sized and shaped to provide a space between the user and the visor 1708 to help avoid contact between the visor 1708 and a chest of the user when a neck of the user is flexed, for example when looking down during procedures.

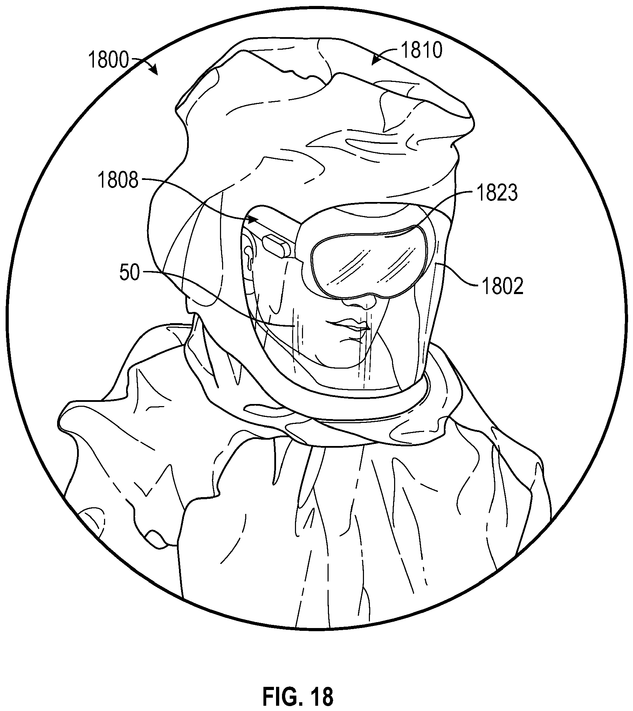

FIG. 18 illustrates a perspective view of a surgical helmet assembly 1800. The surgical helmet assembly 1800 can be similar to the surgical helmet assemblies discussed above. FIG. 18 shows how the surgical helmet assembly 1800 can include a visor that stands off from the MR device. Any of the surgical helmet assemblies discussed above or below can be modified to include the features of the surgical helmet assembly 1800.

More specifically, the surgical helmet assembly 1800 can include a MR device 1802, which can be similar to any of the AR or MR devices discussed above. The MR device 1802 can be securable to a head of the surgeon 50. The surgical helmet assembly 1800 can also include a visor 1808 securable to the MR device 202 and the. The surgical helmet assembly 1800 can also include a hood 1810 connected to and at least partially surrounding the visor 1808.

The visor 1810 can be a transparent or translucent shield configured to provide visibility for the surgeon 50 while limiting transfer of matter between the surgeon 50 and the surgical environment, which can be similar to the visor 1708. The hood 1810 can be a drape or covering connected to the visor 1808. The hood 1810 can be secured to a perimeter of the visor 1808 and can be configured to cover at least a portion of a head of the surgeon 50.

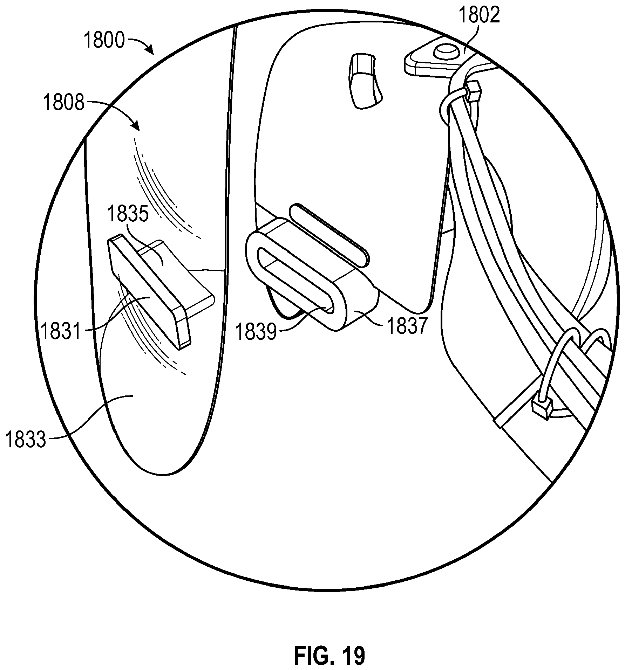

In operation, the surgeon 50 can wear the MR device 1802 with the visor 1808 and hood 1810 attached thereto. Following a procedure or other use of the MR device 1802, the 1808, and the visor 1808 and the hood 1810 can be disconnected from the MR device 1802, such as by disengaging one or more fasteners or clips, as discussed with respect to FIG. 19.

FIG. 19 illustrates an enlarged perspective view of a portion of the surgical helmet assembly 1800. More specifically, FIG. 19 shows that a clip or fastener 1831 can be connected to (or fastened to) a lateral portion 1833 of the visor 1808. The fastener 1831 can be a clip, connector, fastener, or the like. The fastener 1831 can be configured to secure the lateral portion 1833 to the MR device 1802.

The fastener 1831 can include a projection 1835, which can be insertable into a bore 1839 of a MR fastener 1837. The MR fastener 1837 can be a receiver connected to or otherwise secured to the MR device 1802. Insertion of the projection 1835 into the bore 1839 can secure the fastener 1831 to the MR fastener 1837 to secure the lateral portion 1833 of the visor 1808 to the MR device 1802 to secure the visor 1808 to the MR device 1802. Securing of the projection 1835 to the MR fastener 1837 can orient the visor 1808 with respect to the MR device 1802, such as to help create the appropriate gaps and spacing of the visor 1808 with respect to the MR device 1802 and the surgeon 50. Optionally, the visor 1808 and MR device 1802 can include multiple clips, such as one clip on each side or multiple clips on both sides.

The projection 1835 can be secured in the 1839 such as through an interference engagement or other locking or latching mechanism. Optionally, the fastener 1831 can include a magnetic feature on the projection 1835 (or elsewhere) and the bore 1839 can include a complementary magnetic feature in the bore 1839 or elsewhere to allow the fastener 1831 to magnetically (releasably) secure to the MR fastener 1837.

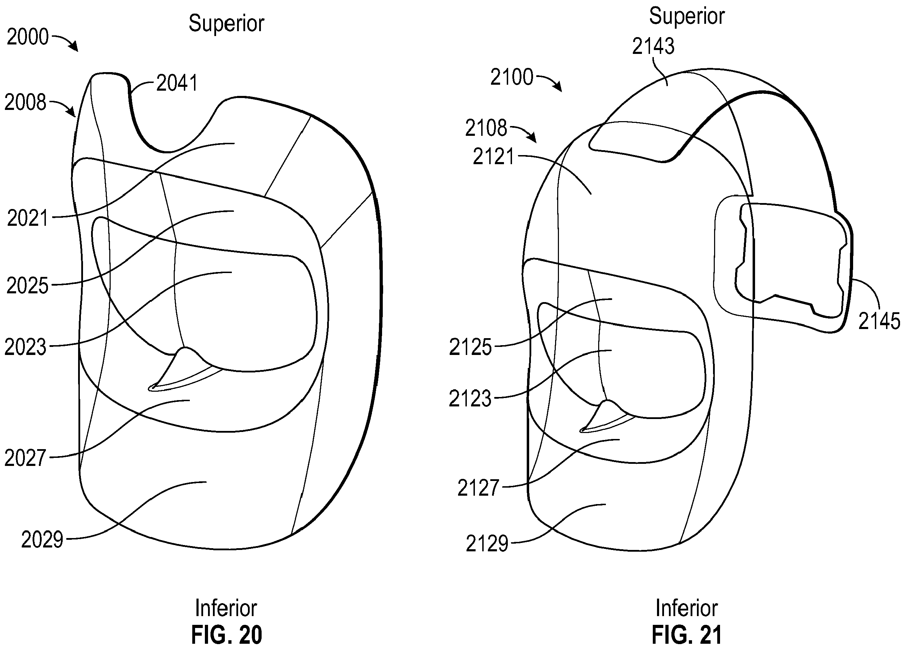

FIG. 20 illustrates an isometric view of a portion of a surgical helmet assembly 2000. The surgical helmet assembly 2000 can be similar to the surgical helmet assemblies discussed above. FIG. 20 shows how the surgical helmet assembly 2000 can include a visor that includes an opening for a light. Any of the surgical helmet assemblies discussed above or below can be modified to include the features of the surgical helmet assembly 2000.

The surgical helmet assembly 2000 can be similar to the surgical helmet assembly 1700 in that the surgical helmet assembly 2000 can include a visor 2008 including an upper portion 2021, a sensor portion 2023, an angled portion 2025, a lower angled portion 2027, and a lower portion 2029. The portions of the surgical helmet assembly 2000 can be connected and can function or operate similarly to the portion of the surgical helmet assembly 1700.

The upper portion 2021 can also include an opening 2041 extending therethrough. The opening 2041 can be a cutout (as shown in FIG. 20) and can also be a notch, bore, or the like. The opening 2041 can be configured to provide user access to a light located within the visor 2008 and a surgical hood. For example, a lens of a light can extend through the opening. Also, the opening 2041 can provide access to an adjustment mechanism of the light, such as an orientation adjustment or an intensity adjustment. In this way, the visor 2008 can provide access to the user to adjust the light without removing the surgical helmet assembly 2000.

FIG. 21 illustrates an isometric view of a portion of a surgical helmet assembly 2100. The surgical helmet assembly 2100 can be similar to the surgical helmet assemblies discussed above. FIG. 21 shows how the surgical helmet assembly 2100 can include a visor that includes a support for supporting a toga or hood above a head of the user. Any of the surgical helmet assemblies discussed above or below can be modified to include the features of the surgical helmet assembly 2100.

The surgical helmet assembly 2100 can be similar to the surgical helmet assembly 1700 in that the surgical helmet assembly 2100 can include a visor 2108 including an upper portion 2121, a sensor portion 2123, an angled portion 2125, a lower angled portion 2127, and a lower portion 2129. The portions of the surgical helmet assembly 2100 can be connected and can function or operate similarly to the portion of the surgical helmet assembly 1700.

The surgical helmet assembly 2100 can also include a support 2143 connected to the visor 2108 such as to the upper portion 2121. The support 2143 can extend superiorly and posteriorly from the upper portion 2121 to a posterior portion of the head of the user, the support configured to support the surgical hood off the head of the user. The support 2143 can be configured to engage and support a hood or toga of the surgical helmet assembly 2100 such as above a head of the user.

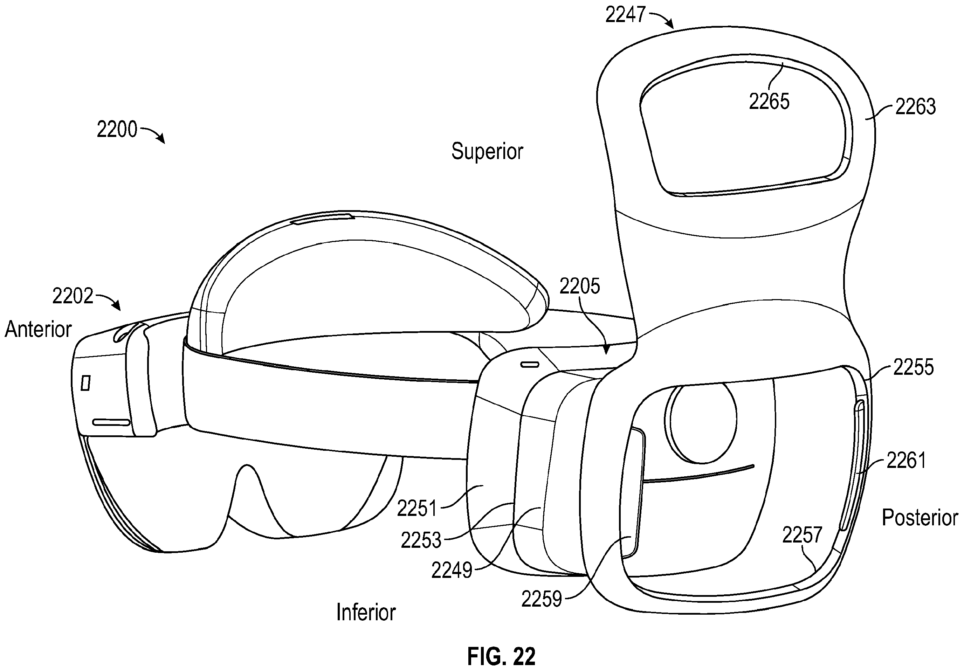

The support 2143 can further extend posteriorly and superiorly such that the support 2143 connect to a rear support 2145. The rear support 2145 can be configured to engage a head of the user for additional support. Optionally, the rear support 2145 can be securable to a posterior portion of the MR device, such as the battery pack 505. The rear support 2145 can operate similarly to the support of FIG. 22, discussed below.

FIG. 22 illustrates an isometric view of a portion of a surgical helmet assembly 2200. The surgical helmet assembly 2200 can be similar to the surgical helmet assemblies discussed above. FIG. 22 shows how the surgical helmet assembly 2200 can include a support connectable to the MR device for supporting a toga or hood above a head of the user. Any of the surgical helmet assemblies discussed above or below can be modified to include the features of the surgical helmet assembly 2200.

The surgical helmet assembly 2200 can include a MR device 2202 including a battery pack 2205 located at a posterior portion of the surgical helmet assembly 2200. The surgical helmet assembly 2200 can also include a support 2247 releasably connectable to the MR device 2202 such as to the battery pack 2205. The battery pack 2205 can include a battery 2249 and a cushion 2251 defining a gap or slot 2253 therebetween. The cushion 2251 can be configured to engage a posterior portion of the surgeon 50.

The support 2247 can include a ring 2255 located at an inferior portion thereof. The ring 2255 can define an opening sized to at least partially surround a rear portion of the MR device 2202, such as the battery 2249 or the cushion 2251. The ring 2255 can include tabs 2259 and 2261. The tab 2259 (or first projection) can extend inward from the ring 2255 and can be insertable at least partially into the slot 2253 to secure the ring 2255 to the battery pack 2205. Similarly, the tab 2261 (or second projection) can extend inward from the ring 2255 opposite the tab 2259 and can be insertable at least partially into a slot (opposite slot 2253) to secure the ring 2255 to, together with the tabs 2259, secure the ring 2255 (and therefore the support 2247) to the battery pack 2205 of the MR device 2202.

The support 2247 can also include an upper portion 2263 connected to the ring 2255 and engageable with the surgical hood to hold the surgical hood above the head of the user once the support 2247 is secured to the MR device 2202. Optionally, the upper portion 2263 can define an opening 2265 that can, at least in part, form a handle that can be user-graspable to allow a user (e.g., the surgeon 50) to carry the surgical helmet assembly 2200 when the support 2247 is connected to the MR device 2202.

### NOTES AND EXAMPLES

The following, non-limiting examples, detail certain aspects of the present subject matter to solve the challenges and provide the benefits discussed herein, among others.

Example 1 is a surgical helmet assembly securable to a mixed reality device, the assembly comprising: an inner frame securable to the mixed reality device; an outer frame releasably securable to the inner frame, at least a portion of a lens of the mixed reality device exposed through the inner frame and the outer frame; and a surgical hood connected to the outer frame and configured to cover at least a portion of a head of a user.

In Example 2, the subject matter of Example 1 optionally includes wherein the inner frame includes a first arm and a second arm each laterally extending from the inner frame, and wherein the outer frame includes a third arm and a fourth arm extending from the outer frame, the first arm securable to the third arm and the second arm securable to the fourth arm to secure the inner frame to the outer frame.

In Example 3, the subject matter of Example 2 optionally includes a gasket engageable with the inner frame and the outer frame to form a seal between the inner frame to the outer frame when the first and second arms are secured to the third and fourth arms.

In Example 4, the subject matter of any one or more of Examples 1-3 optionally include a visor secured to an outer portion of the outer frame and to the surgical hood.

In Example 5, the subject matter of Example 4 optionally includes wherein an outer surface of the lens is exposed to an environment.

In Example 6, the subject matter of any one or more of Examples 1-5 optionally include a pillar at least partially insertable into a portion of the mixed reality device and engageable with the inner frame to secure the inner frame to mixed reality device.

In Example 7, the subject matter of Example 6 optionally includes wherein the inner frame includes a top cover engageable with a top portion of the mixed reality device, the pillar securable to the inner frame to secure the inner frame to the mixed reality device.

In Example 8, the subject matter of Example 7 optionally includes wherein the outer frame includes a visor connected to the hood, the visor securable to the inner frame to secure the hood and the visor to the inner frame.

In Example 9, the subject matter of any one or more of Examples 1-8 optionally include wherein the lens of the mixed reality device is exposed to an environment through the inner frame and the outer frame.

In Example 10, the subject matter of any one or more of Examples 1-9 optionally include a cooling system connected to the inner frame and connectable to the mixed reality device, and locatable within the surgical hood, the cooling system operable to deliver conditioned air to the inner frame, the mixed reality device, and the surgical hood.

Example 11 is a surgical helmet assembly securable to a mixed reality device, the assembly comprising: an inner frame securable to the mixed reality device; an outer frame releasably securable to the inner frame; a visor releasably securable to the inner frame, at least a portion of a lens of the mixed reality device exposed through the visor and the outer frame; and a surgical hood connected to the visor and configured to cover at least a portion of a head of a user and to enclose at least a portion of the mixed reality device.

In Example 12, the subject matter of Example 11 optionally includes wherein the inner frame includes a first arm and a second arm each laterally extending from the inner frame, and wherein the outer frame includes a third arm and a fourth arm extending from the outer frame, the first arm securable to the third arm and the second arm securable to the fourth arm to secure the inner frame to the outer frame.

In Example 13, the subject matter of Example 12 optionally includes a gasket engageable with the inner frame and the outer frame to form a seal between the inner frame to the outer frame when the first and second arms are secured to the third and fourth arms.

In Example 14, the subject matter of Example 13 optionally includes wherein an outer surface of the lens is exposed to an environment.

In Example 15, the subject matter of any one or more of Examples 11-14 optionally include wherein the lens of the mixed reality device is exposed to an environment through the inner frame and the outer frame.

In Example 16, the subject matter of any one or more of Examples 11-15 optionally include a cooling system connected to the inner frame and connectable to the mixed reality device, and locatable within the surgical hood, the cooling system operable to deliver conditioned air to the inner frame, the mixed reality device, and the surgical hood.

Example 17 is a surgical helmet assembly securable to a mixed reality device, the assembly comprising: an inner frame securable to the mixed reality device; a visor releasably securable to the inner frame, at least a portion of a lens of the mixed reality device exposed through the visor and the inner frame; and a surgical hood connected to the visor and configured to cover at least a portion of a head of a user and to enclose at least a portion of the mixed reality device.

In Example 18, the subject matter of Example 17 optionally includes a pillar at least partially insertable into a portion of the mixed reality device and engageable with the inner frame to secure the inner frame to mixed reality device.

In Example 19, the subject matter of Example 18 optionally includes wherein the inner frame includes a top cover engageable with a top portion of the mixed reality device, the pillar securable to the inner frame to secure the inner frame to the mixed reality device.

In Example 20, the subject matter of any one or more of Examples 17-19 optionally include a cooling system connected to the inner frame and connectable to the mixed reality device, and locatable within the surgical hood, the cooling system operable to deliver conditioned air to the inner frame, the mixed reality device, and the surgical hood.

Example 21 is a surgical helmet assembly securable to a mixed reality device, the assembly comprising: a first frame securable to the mixed reality device; a second frame releasably securable to the first frame, at least a portion of the mixed reality device exposed through the first frame and the second frame; and a fastener releasably securable to the first frame to secure the second frame to the first frame.

In Example 22, the subject matter of Example 21 optionally includes wherein the portion of the mixed reality device exposed through the first frame and the second frame is a sensor unit.

In Example 23, the subject matter of any one or more of Examples 21-22 optionally include wherein the first frame forms a circular opening to expose the portion of the mixed reality device.

In Example 24, the subject matter of Example 23 optionally includes wherein the first frame is a cylindrical frame secured to the mixed reality device.

In Example 25, the subject matter of Example 24 optionally includes a gasket engageable with the first frame and the fastener to form a seal between the first frame and the second frame when the fastener is secured to the first frame.

In Example 26, the subject matter of Example 25 optionally includes wherein the gasket is connected to an outer portion of the cylindrical frame.

In Example 27, the subject matter of Example 26 optionally includes wherein the fastener is threadably securable to the cylindrical frame.

Example 28 is a surgical helmet assembly securable to a mixed reality device, the assembly comprising: a visor releasably securable to the mixed reality device, at least a portion of a lens of the mixed reality device spaced away from the visor; and a surgical hood connected to the visor and configured to cover at least a portion of a head of a user and to enclose at least a portion of the mixed reality device.

In Example 29, the subject matter of Example 28 optionally includes wherein the visor is shaped to form an air gap between a top portion of a user's head and the surgical hood.

In Example 30, the subject matter of Example 29 optionally includes wherein the visor includes an upper portion connected to a sensor portion, the upper portion curved from anterior to posterior as the visor extends superiorly from the sensor portion to form the air gap.

In Example 31, the subject matter of Example 30 optionally includes wherein the visor is engageable with the mixed reality device to position sensors of the mixed reality device away from the sensor portion of the visor by a distance of a sensor gap.

In Example 32, the subject matter of Example 31 optionally includes wherein the sensor gap is one millimeter or less.

In Example 33, the subject matter of any one or more of Examples 30-32 optionally include wherein the visor includes an angled portion connecting the sensor portion and the upper portion, the angled portion angled with respect to the sensor portion to transmit light therethrough.

In Example 34, the subject matter of Example 33 optionally includes wherein the angled portion is configured to direct light away from the sensor portion.

In Example 35, the subject matter of any one or more of Examples 33-34 optionally include wherein the upper portion includes an opening extending therethrough and configured to provide user access to a light located at least partially within the visor and the surgical hood and connected to the surgical helmet assembly.

In Example 36, the subject matter of any one or more of Examples 30-35 optionally include a support extending from the upper portion and configured to extend to a posterior portion of the head of the user, the support configured to support the surgical hood off the head of the user.

In Example 37, the subject matter of any one or more of Examples 28-36 optionally include wherein the visor includes a bottom portion configured to form a gap between a face of the user and the bottom portion.

In Example 38, the subject matter of any one or more of Examples 28-37 optionally include a fastener connected to a lateral portion of the visor and securable to a receiver of the mixed reality device to orient the visor with respect to the mixed reality device.

In Example 39, the subject matter of any one or more of Examples 28-38 optionally include millimeters.

In Example 40, the subject matter of Example 39 optionally includes millimeters.

In Example 41, the subject matter of any one or more of Examples 28-40 optionally include a support connectable to a rear portion of the mixed reality device, the support engageable with the surgical hood to hold the surgical hood above the head of the user.

In Example 42, the subject matter of Example 41 optionally includes wherein the support defines a ring to at least partially surround the rear portion of the mixed reality device.

In Example 43, the subject matter of Example 42 optionally includes wherein the support defines a first projection extending inward from the ring, the first projection insertable at least partially into the rear portion of the mixed reality device to secure the ring to the rear portion of the mixed reality device.

In Example 44, the subject matter of Example 43 optionally includes wherein the support defines a second projection extending inward from the ring opposite the first projection, the second projection insertable at least partially into the rear portion of the mixed reality device opposite the first projection to, together with the first projection, secure the ring to the rear portion of the mixed reality device.

In Example 45, the apparatuses, systems, or methods of any one or any combination of Examples 1 - 44 can optionally be configured such that all elements or options recited are available to use or select from.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) can be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features can be grouped together to streamline the disclosure. This should not be interpreted as intending that an unclaimed disclosed feature is essential to any claim. Rather, inventive subject matter can lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. A surgical helmet assembly securable to a mixed reality device, the assembly comprising:
an inner frame securable to the mixed reality device;
an outer frame releasably securable to the inner frame, at least a portion of a lens of the mixed reality device exposed through the inner frame and the outer frame; and
a surgical hood connected to the outer frame and configured to cover at least a portion of a head of a user.

2. The assembly of claim 1, wherein the inner frame includes a first arm and a second arm each laterally extending from the inner frame, and wherein the outer frame includes a third arm and a fourth arm extending from the outer frame, the first arm securable to the third arm and the second arm securable to the fourth arm to secure the inner frame to the outer frame.

3. The assembly of claim 2, further comprising:
a gasket engageable with the inner frame and the outer frame to form a seal between the inner frame to the outer frame when the first and second arms are secured to the third and fourth arms.

4. The assembly of any of claims 1-3, further comprising:
a visor secured to an outer portion of the outer frame and to the surgical hood.

5. The assembly of claim 4, wherein an outer surface of the lens is exposed to an environment.

6. The assembly of any of claims 1-5, further comprising:
a pillar at least partially insertable into a portion of the mixed reality device and engageable with the inner frame to secure the inner frame to mixed reality device.

7. The assembly of claim 6, wherein the inner frame includes a top cover engageable with a top portion of the mixed reality device, the pillar securable to the inner frame to secure the inner frame to the mixed reality device.

8. The assembly of claim 7, wherein the outer frame includes a visor connected to the hood, the visor securable to the inner frame to secure the hood and the visor to the inner frame.

9. The assembly of any of claims 1-8, wherein the lens of the mixed reality device is exposed to an environment through the inner frame and the outer frame.

10. The assembly of any of claims 1-9, further comprising:
a cooling system connected to the inner frame and connectable to the mixed reality device, and locatable within the surgical hood, the cooling system operable to deliver conditioned air to the inner frame, the mixed reality device, and the surgical hood.
